# EUROPÄISCHE PATENTSCHRIFT

(11) **EP 0 643 062 B1**
(45) Veröffentlichungstag und Bekanntmachung des Hinweises auf die Patenterteilung: **17.08.2005**
(21) Anmeldenummer: 94810503.6
(22) Anmeldetag: 01.09.1994
(51) Int. Cl.: C07D 519/00, B41M 5/145, C07D 493/10

(54) **Bislactone**
Bislactones
Bislactones

(30) Priorität: 09.09.1993 CH 270493
(43) Veröffentlichungstag der Anmeldung: 15.03.1995
(73) Patentinhaber: Ciba Specialty Chemicals Holding Inc., 4057 Basel (CH)
(72) Erfinder: Zink, Rudolf, CH-4106 Therwil (CH)

(56) Entgegenhaltungen:
- EP-A- 0 561 738
- FR-A- 2 187 853
- US-A- 3 715 226
- US-A- 3 925 416
- US-A- 4 012 419
- CHEMICAL ABSTRACTS, vol. 111, no. 18, 30. Oktober 1989, Columbus, Ohio, US; abstract no. 164340x, J. FUKUSHIGE ET AL. 'Recording material containing fluorane derivative as electron-donating dye.' Seite 730 ;Spalte 1 ; & JP-A-01 077 574 (FUJI PHOTO FILM CO., LTD.) 23. März 1989
- z. yoshida et al.: "Chemistry of Functional Dyes" Mita Press , Tokyo, Japan
- w. Herbst et al.: "Industrielle organische Pigmente" VCH Verlagsgesellschaft , Weinheim, DE

## Beschreibung

Die vorliegende Erfindung betrifft chromogene Bislactonverbindungen mit verbesserter Sublimationsechtheit und Migrationsstabilität, Verfahren zu ihrer Herstellung, ihre Verwendung als Farbbildner in druck- oder wärmeempfindlichen Aufzeichnungsmaterialien sowie die Herstellung dieser Aufzeichnungsmaterialien.

Die Bislactonverbindungen entsprechen der allgemeinen Formel (1) 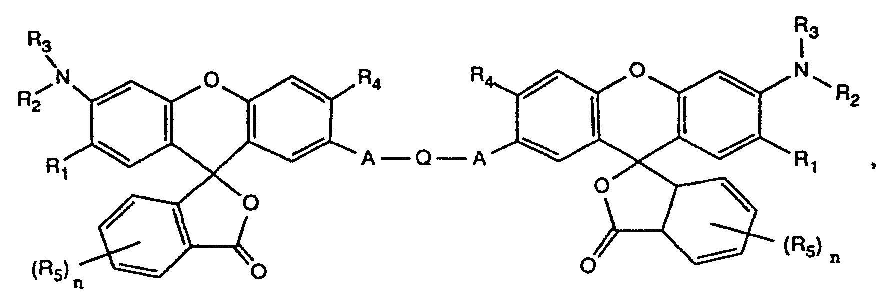 worin
- R₁: Wasserstoff oder C₁-C₄-Alkyl;
- R₂ und R₃: unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl; oder
- R₂ und R₃: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
- R₄: Wasserstoff oder C₁-C₄-Alkyl;
- R₅: Halogen; Nitro; C₁-C₄-Alkyl; C₁-C₄-Halogenalkyl; Amino; mono-C₁-C₄-Alkylamino; di-C₁-C₄-Alkylamino; oder COR₆
- n: 0; 1; 2; 3; oder 4;
- R₆: Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl oder C₁-C₄-Alkoxy substituiertes Phenyl; Phenyl-C₁-C₄-Alkyl oder Phenyl-C₁-C₄-Alkoxy,
- A: -(SO)O- oder -(CO)O-, welche über S bzw. C an das Fluorangerüst gebunden sind, und
- Q: einen gesättigten aliphatischen Rest, der durch Halogen substituiert sein kann, einen ungesättigten aliphatischen Rest oder einen gesättigten oder ungesättigten aliphatischen Rest, der durch Sauerstoffatome unterbrochen sein kann, wobei die Molekulargewichte dieser Reste zwischen 28 und 150 liegen, oder einen cycloaliphatischen oder araliphatischen Rest mit höchstens 10 Kohlenstoffatomen oder einen 2-Buten-1,4-diyl- oder Ethoxycarbonylethylenrest bedeutet.

In der Literatur werden die einzelnen Substituentenposititionen im Fluoranring unterschiedlich numeriert. In der vorliegenden Anmeldung wird die folgende Numerierung verwendet:

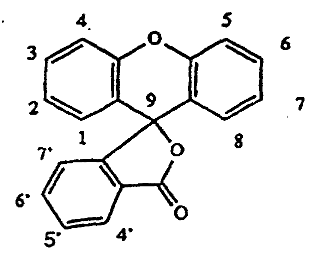

Im Rahmen der vorstehenden Definition haben die jeweiligen Substituenten insbesondere die folgenden Bedeutungen:
Halogen ist Fluor, Chlor oder Brom, insbesondere Fluor oder Chlor.
Alkyl bedeutet im Rahmen der jeweiligen Definition geradkettiges oder verzweigtes Alkyl. Beispiele solcher Alkylreste sind Methyl, Ethyl, n-Propyl, Isopropyl, n-Butyl, 1-Methylbutyl, sek.Butyl, tert.Butyl, n-Pentyl, Amyl, Isoamyl, n-Hexyl, 2-Ethyl-hexyl, n-Heptyl, n-Octyl, Isooctyl, 1,1,3,3-Tetramethylbutyl.

Beispiele für Halogenalkyl sind insbesondere die C₁-C₂-Halogenalkylreste, wie Trichlormethyl, Trifluormethyl, Dichlorfluormethyl, Difluorchlormethyl, Perchlorethyl, 1,1,2,2-Tetrachlorethyl, 1,1,2,2-Tetrafluorethyl, 2,2,2-Trichlorethyl. R₆ als C₁-C₈-Halogenalkyl sind insbesondere die zuvor genannten Halogenalkyle aber auch Alkylradikale, bei denen alle oder zumindest der überwiegende Teil der C-H Bindungen durch C-Cl oder C-F ersetzt ist.

Alkoxy ist insbesondere Methoxy, Ethoxy, n-Propoxy, Isopropoxy, n-Butoxy, Isobutoxy, sek.Butoxy und tert.Butoxy, C₁-C₄-Alkoxy-C₁-C₄Alkyl insbesondere Methoxymethyl, Methoxyethyl, Ethoxymethyl oder Ethoxyethyl.

Mono-C₁-C₅-Alkylamino ist insbesondere Methylamino, Ethylamino, Propylamino, Butylamino und Pentylamino. Di-C₁-C₅-Alkylamino umfaßt sowohl die gemischten wie auch die gleichnamig substituierten Radikale, wie Methylethylamino, Dimehtylamino, Diethylamino, Methylpropylamino, Methylbutylamino, Di-n-propylamino, Di-isopropylamino, Di-n-butylamino und Di-n-pentylamino etc..

In Phenyl-C₁-C₄-Alkyl und Phenyl-C₁-C₄-Alkoxy kann der Phenylrest über eine geradkettige oder verzweigte Alkyl- bzw. Alkoxykette gebunden sein. Bevorzugt ist Phenethyl, Benzyl und Phenylmethoxy.

Der Phenylrest in Phenyl-C₁-C₄-Alkyl, Phenyl-C₁-C₄-Alkoxy bzw. Phenyl ist vorzugsweise unsubstituiert oder bis zu dreifach gleich oder verschieden durch die genannten Substituenten substituiert.

Beispiele für C₃-C₅-Alkenyl sind Allyl, 1-Propenyl oder 2-Pentenyl, Isopropenyl oder 2-Butenyl. Allyl ist bevorzugt. C₄-C₇-Cycloalkyl bedeutet Cyclobutyl, Cyclopentyl, Cyclohexyl oder Cycloheptyl, wobei Cyclohexyl im Vordergrund des Interesses steht.

Q stellt in der Bedeutung eines aliphatischen Restes insbesondere eine Alkylengruppe dar, die gegebenenfalls durch Halogenatome, besonders Chlor, substituiert ist. Die Alkylengruppe kann 2 bis 8 Kohlenstoffatome enthalten und geradkettig oder verzweigt sein. Die Alkylengruppe weist vorzugsweise 2 bis 5 Kohlenstoffatome auf. Es handelt sich beispielsweise um die -CH₂CH₂-, 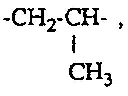 -CH₂CH₂CH₂-, -CH₂CH₂CH₂CH₂-, 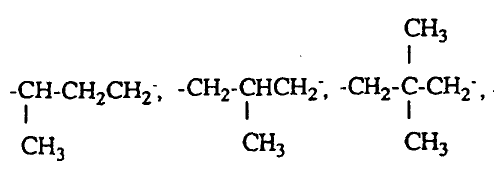 -CH₂(CH₂)₄CH₂- oder -CH₂(CH₂)₆CH₂-Gruppe.

Q kann ausserdem eine der folgenden Gruppen darstellen:

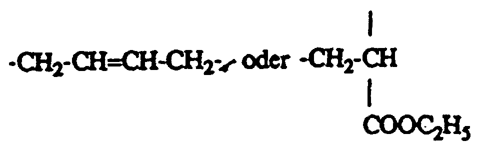

Der aliphatische Kohlenwasserstoffrest kann durch Sauentoffatome unterbrochen sein und somit den Rest eines Polyalkylenglykols, wie z.B. Polyethylenglykols, Polypropylenglykols oder Polybutylenglykols darstellm Dabei bedeutet Q vorteilhafterweise den Rest der Formel

(1a) -CH₂CH₂O CH₂CH₂-

oder

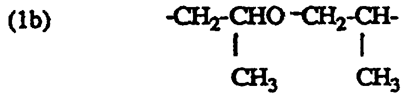

Als cycloaliphatischer Rest bedeutet Q z.B. die 1,2-Cyclopentylengruppe, die 1,2-Cyclohexylengruppe, die 1,3-Cyclohexylengruppe, 1,4-Cyclohexylengruppe oder 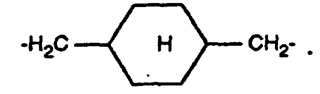 Diese cycloaliphatischen Reste können eine oder zwei Methylengruppen aufweisen.

Als araliphatischer Rest kann Q beispielsweise 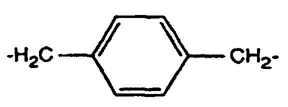 oder 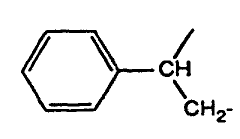 darstellen.

Bevorzugt sind Bislactone der Formel (1), worin
- R₁: Wasserstoff oder Methyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten Pyrrolidin- oder Piperidin-Ring;
- R₄: Wasserstoff oder Methyl;
- n: 0; 1; 2; 3; oder 4;
- R₅: Halogen; Nitro; C₁-C₄-Alkyl; C₁-C₄-Halogenalkyl; mono-C₁-C₄-Alkylamino; di-C₁-C₄-Alkylamino; oder COR₆;
- A: -(SO)O- oder -(CO)O- welche über S bzw. C an das Fluorangerüst gebunden sind, und
- Q: einen gesättigten oder ungesättigten aliphatischen Rest mit einem Molekulargewicht von 28 bis 150 oder einen cycloaliphatischen oder araliphatischen Rest mit höchstens 10 Kohlenstoffatomen,
bedeutet.

Ganz besonders bevorzugt sind Bislactone der Formel (1) worin Q einen gesättigten oder ungesättigten aliphatischen oder cycloaliphatischen Rest, insbesondere einen C₂-C₄-Alkylenrest und vor allem Butylen oder Ethylen bedeutet.

Hervorzuheben sind Verbindungen der Formel (1), bei denen n 0 bedeutet.

Ganz besonders wichtige Bislactonverbindungen entsprechen der Formel (2) 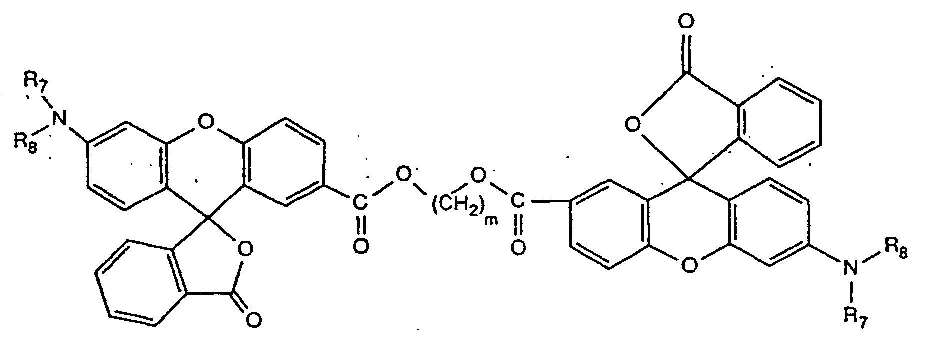 worin
R₇ und R₈ unabhängig voneinander C₁-C₅-Alkyl und
m 2 bis 4
bedeuten.

Die Verbindungen der Formel (1) bzw. (2) sind neu. Das Verfahren zur Herstellung dieser Verbindungen erfolgt nach an sich bekannten Methoden. Es ist dadurch gekennzeichnet, dass man 2 Mol der Verbindung der Formel 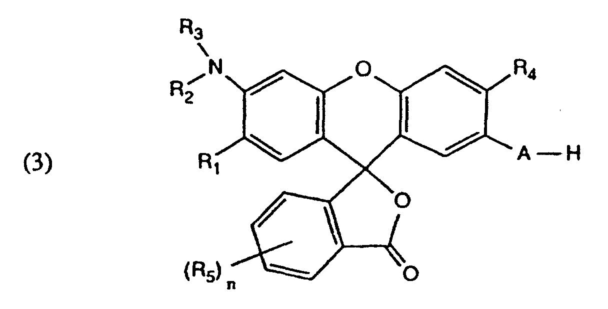 mit 1 Mol einer Dihalogenverbindung der Formel

(4) Hal-Q-Hal

umsetzt, worin
R₁, R₂, R₃, R₄, R₅, A, Q und n die in Formel (1) angegebene Bedeutung haben und Hal Halogen bedeutet.

Halogen bedeutet dabei Fluor, Chlor und vorzugsweise Brom.

Das Verfahren zur Herstellung der Verbindungen der Formel (1) stellt einen weiteren Erfindungsgegenstand dar.

Die Ausgangsverbindungen der Formel (3) sind z.T. aus FR-A-2 187 853 bekannt. Die Herstellung dieser Verbindungen erfolgt z.B. durch die Umsetzung eines Benzophenons der Formel (5) mit einem Phenol oder Phenolether der Formel (6) 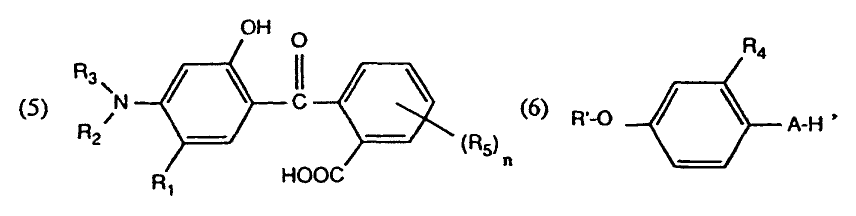 worin die Reste R₁ bis R₅, A und n wie zuvor definiert sind und
R' Wasserstoff oder C₁-C₄-Alkyl bedeutet,
bei Temperaturen von vorzugsweise 0 bis 70° C, in 50 bis 100 %iger Schwefelsäure, Kondensation zu einem Phthalid der Formel 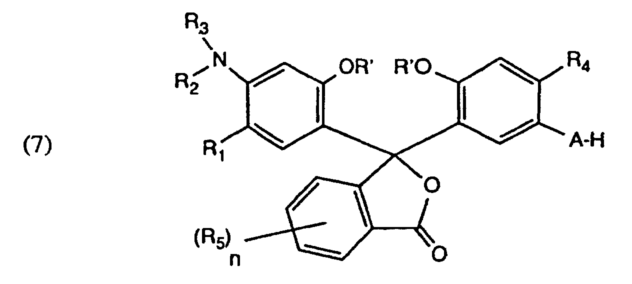 und anschliessender Cyclisierung bei Temperaturen von 20 bis 100° C zu einer Verbindung der Formel (3).

Die Verbindungen der Formel (1) sind normalerweise farblos oder höchstens schwach gefärbt. Wenn diese sublimationsechten Farbbildner mit einem vorzugsweise sauren Entwickler, d.h. einem Elektronenakzeptor, in Kontakt gebracht werden, so ergeben sie je nach der Bedeutung der Substituenten R₁ bis R₆ und dem verwendeten Entwickler intensiv orange oder rote Farbtöne. Die Verbindungen der Formel (1) sind auch sehr wertvoll im Gemisch mit einem oder mehreren anderen bekannten Farbbildnern, wie z.B. 3,3-(Bisaminophenyl-)-phthaliden, wie CVL, 3-Indolyl-3-aminophenyl- aza- oder -diazaphthaliden, (3,3-Bis-indolyl-)-phthaliden, 3-Aminofluoranen, 3,7-Diaminofluoranen, 3,7-Diamino-6-methyl-fluoranen, 3,6-Bisalkoxyfluoranen, 3,6-Bisdiarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um blaue, marineblaue, graue oder schwarze Färbungen zu ergeben.

Bevorzugt als Mischpartner sind Fabbildner, die eine schwarze Nuance ergeben, wie insbesondere 3,7-Diaminofluorane.

Die Verbindungen der Formel (1) werden zum Erzielen von marineblauen, grauen oder schwarzen Färbungen in einem für die gewünschte Farbtiefe geeigneten Mischungsverhältnis mit den anderen Farbbildnern eingesetzt. Die Mischungsverhältnisse werden auch durch den bei der Farbreaktion verwendeten Entwickler beeinflußt. Sie können in einfachen Serienuntersuchungen festgestellt werden. Vorzugsweise werden in den Farbmischungen 10 bis 70 % des Bislactons der Formel (1) (Angaben in % bezogen auf die Mengen der eingesetzten Farbbildner) eingesetzt. Besonders bevorzugt sind etwa 20 bis 50 %. Derartige Mischungen kommen sowohl für Reaktionsdurchschreibepapiere als auch für Thermodruckpapiere in Frage. Im folgenden umfaßt der Begriff "Farbbildner" die Farbbildner der Formel (1), wie auch deren Mischungen.

Die Verbindungen der Formel (1) zeigen sowohl auf aktivierten Tonen, wie auch auf phenolischen Unterlagen eine ausgezeichnete Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH-stabil, in den Kapselölen hervorragend löslich sowie sublimations- als auch migrationsecht sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, das mindestens einen Farbbildner der Formel (1) gelöst in einem organischen Lösungsmittel und einen Elektronenakzeptor als Entwickler enthält.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxid, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethylbenzyl)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein. Die Zinksalicylate sind z.B. in EP-A-0,181,283 oder DE-A-2,242,250 beschrieben.

Die Entwickler können zusätzlich auch im Gemisch mit an sich unreaktiven oder wenig reaktiven Pigmenten oder weiteren Hilfsstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxid, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmäßig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes, mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester wie z.B. Tricresylphosphat, Di-n-butylphthalat, Dioctylphthalat, Trichlorethylphosphat, aromatische Ether wie Benzylphenylether, Kohlenwasserstofföle, wie Paraffin oder Kerosin, aromatische Kohlenwasserstoffe, z.B. mit Isopropyl, Isobutyl, sek.Butyl oder tert.Butyl alkylierte Derivate von Diphenyl, Naphthalin oder Terphenyl, Dibenzyltoluol; partiell hydriertes Terphenyl, mono-, bis- oder tetra-C₁-C₃-alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Phthalide dadurch aus, dass sie gut löslich sind und eine pH-Beständigkeit über einen weiten Bereich, z.B. von 4 bis 10, zeigen.

Die Kapselwände können durch Koazervationskräfte gleichmäßig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formel (1) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Mit Vorteil können die erfindungsgemässen Farbbildner der Formel (1) als Mischungskomponenten in mikroverkapselte Farbbildnersystemen eingesetzt werden. Dabei ist die Sublimationsechtheit der erfindungsgemässen Bislactone der Formel (1) von besonderem Vorteil. Die aus dem Stand der Technik bekannten Bislactone neigen, wie eingangs erwähnt, eher zur Sublimation bzw. Migration als die anderen in derartigen Mischungen eingesetzten Farbbildner. Das Mischungsverhältnis muß deshalb den Eigenschaften der am ehesten sublimierenden Komponente solcher Mischungen Rechnung tragen. Demgegenüber entspricht die Sublimationsechtheit der erfindungsgemässen Verbindungen denen der übrigen Mischungskomponenten. Die daraus resultierenden Vorteile liegen auf der Hand: bei der Auswahl der Mischungskomponenten muß keine besondere Rücksicht auf leichter sublimierbare Bestandteile des Farbbildnersystems genommen werden. Die Verbesserung der Migrationsstabilität in den erfindungsgemässen Farbmischungen wirkt sich insbesondere vorteilhaft auf das entwickelte Bild aus. Auch nach längerem Lagern des Kopiermaterials bleibt das Bild an seinen Rändern scharf und verschwimmt nicht.

Bevorzugt wird eine Anordnung, bei der der eingekapselte Farbbildner in Form einer Schicht auf der Rückseite eines Übertragungsblattes und der Elektronenakzeptor (Farbentwickler) in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Vorzugsweise weist das Durchschreibematerial auch eine kapselfreie, den Farbbildner enthaltende Schicht und eine farbentwickelnde Schicht auf, die als Farbentwickler mindestens ein anorganisches Metallsalz eines mehrwertigen Metalls, vor allem Halogenide oder Nitrate, wie z.B. Zinkchlorid, Zinnchlorid, Zinknitrat oder deren Gemische enthält.

Die Verbindungen der Formel (1) werden vorzugsweise als Farbbildner in einem thermoreaktiven Aufzeichnungsmaterial verwendet. Dieses enthält in der Regel mindestens einen Schichtträger, einen oder mehrere Farbbildner, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Mit Vorteil können die erfindungsgemässen Farbbildner der Formel (1) als Mischungskomponenten in thermoreaktiven Farbbildnersystemen eingesetzt werden. Dabei ist die Lagerstabilität (heiss und/oder feucht) und die geringe Hintergrundverfärbung der eindungsgemässen Bislactone von besonderem Vorteil.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Die Schicht bzw.die Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren wie sie in druckempfindlichen Papieren verwendet werden. Beispiele für Entwickler sind die bereits erwähnten Tonminerale und Phenolharze oder auch phenolische Verbindungen, wie sie beispielsweise in der DE-OS-1,251,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäuremethyl- oder -benzylester, 4-Hydroxydiphenylsulfon, 4'-Hydroxy-4-methyldiphenylsulfon, 4'-Hydroxy-4-isopropoxydiphenylsulfon, 4,4'-Cyclo-hexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Farbbildner und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol verwendet werden. Die bevorzugte Anordnung ist jedoch diejenige, bei der der Farbbildner und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten z.B. Talk, Titandioxid, Zinkoxid, Aluminiumhydroxid, Calciumcarbonat (z.B. Kreide), Tone oder auch organische Pigmente, wie z.B. Harnstoff-Formaldehydpolymerisate enthalten. Um die Farbbildung nur innerhalb eines begrenzten Temperaturbereiches zu bewirken, können Substanzen wie Harnstoff, Thioharnstoff, Diphenylthioharnstoff, Acetamid, Acetanilid, Benzolsulfanilid, Stearinsäureamid, Bis-stearoylethylendiamid, Phthalsäureanhydrid, Metallstearate, wie z.B. Zinkstearat, Phthalsäurenitril, Dimethylterephthalat, Dibenzyltherephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formel (1) ist die Herstellung eines Farbbildes mittels photohärtbarer Mikrokapseln, wie sie z.B. in der DE-OS-3,247,488 beschrieben sind.

US-A-3,715,226 und EP-A-0561738, die allerdings nur Stand der Technik gemäss Artikel 54(3) darstellt, beschreiben Mono-Fluoranverbindungen, und US-A-3,925,416 beschreibt unter anderem Bis-Fluoranverbindungen, bei denen die beiden Fluoranreste über einen aliphatischen Rest mit zwei Aminogruppen verbunden sind.

Chemistry of Functional Dyes, First International Symposium on Chemistry of Functional Dyes, Mita Press, Tokyo, 1989, 397-400 beschreibt 2,2-8is[4-[6'-(n-cyclohexyl-N-methylamino)-3'-methylspiro[phthalid-3,9'-xanthen]-2'-ylamino]phenyl]propan, d.h. ein Bislacton, in dem zwei Fluoraneinheiten über zwei basische Gruppen -NH-Phenylen-Alkylen-Phenylen-NH- miteinander verbunden sind.

US 4,012,419 beschreibt ebenfalls Bisfluoran-Verbindungen, welche keine zwei -(SO)O- oder -(CO)O- Gruppen als Brückenglieder zwischen den beiden Fluoraneinheiten enthalten.

Die folgenden Beispiele erläutern die Erfindung. Die angegebenen Prozentsätze beziehen sich, wenn nichts anderes angegeben ist, auf das Gewicht.

### Herstellungsbeispiele:

Beispiel 1: 80 g des wasserfeuchten Produktes der Formel 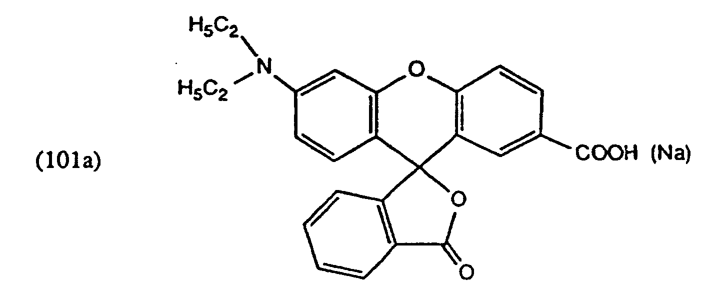 (0,05 Mol in 100 ml Toluol), 10 g Natriumcarbonat und 0,5 g Tetrabutylammoniumbromid werden mit einem Wasserabscheider azeotrop getrocknet. Man gibt 5,4 g (0,025 Mol) 1,4-Dibrombutan hinzu und rührt während 16 Stunden bei 80°C, wobei sich das Produkt der Formel (101) 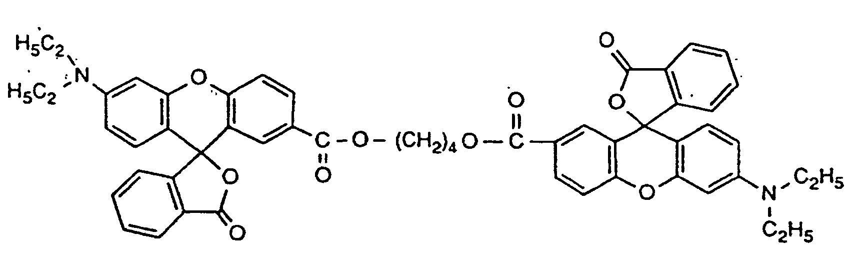 bildet.

Zur Aufarbeitung wird die Toluollösung klärfiltriert, das Toluol abdestilliert und der Rückstand aus einer Mischung von 8 Teilen Isopropanol und 5 Teilen Toluol umkristallisiert.

Man erhält 9 g eines praktisch reinen Produktes mit einem Schmelzpunkt von 105-110°C.

Beispiele 2 bis 8: Analog Beispiel 1 erhält man die in Tabelle 1 aufgeführten Verbindungen:

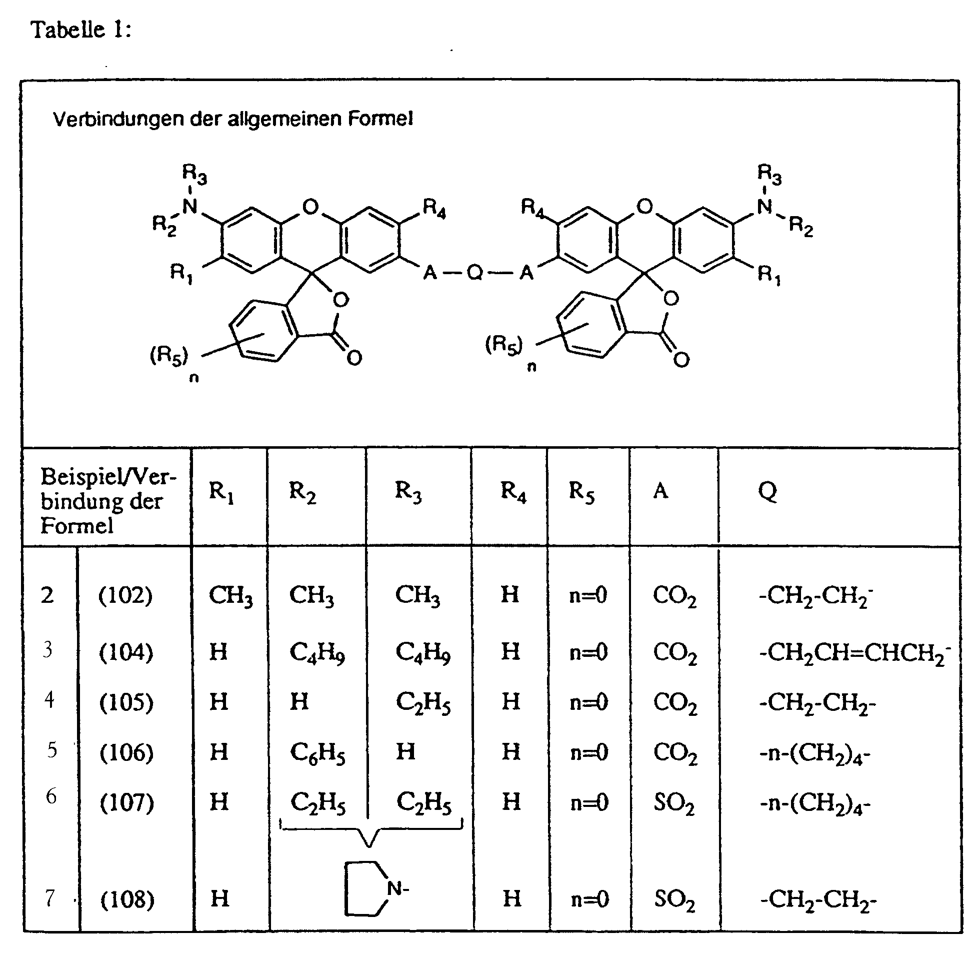

### Anwendungsbeispiele:

Beispiel 8: Eine Lösung von 1 g der Bislactonverbindung der Formel (101) in 80 g Diisopropylnaphthalin und 19 g Kerosin wird in an sich bekannter Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste, den sublimationsechten Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive orange Kopie, die ausgezeichnet licht- und sublimationsecht ist.

Beispiel 9: In einer Kugelmühle werden 32 g 4,4'-Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen, bis die Teilchengrösse ca. 5 µm beträgt. In einer zweiten Kugelmühle werden 2 g der Verbindung der Formel (101), 4 g 3-Diethylamino-7-n-Octylaminofluoran und 3,3 g eines zu 88% hydrolysierten Polyvinylalkohols sowie 60 ml Wasser zu einer Teilchengrösse von ca. 3 µm gemahlen.

Die beiden Dispersionen werden zusammengegeben und mit einem Trockenauftragsgewicht von 0,5 g/m², bezogen auf den Farbbildner, auf ein Papier gestrichen.

Das getrocknete Papier entwickelt beim Beschreiben mit einem handelsüblichen Faksimile-Gerät (®Infotec 6510) eine schwarze Schrift. Das Papier zeigt keine Hintergrundverfärbung.

## Patentansprüche

1. Bislacton der Formel (1) 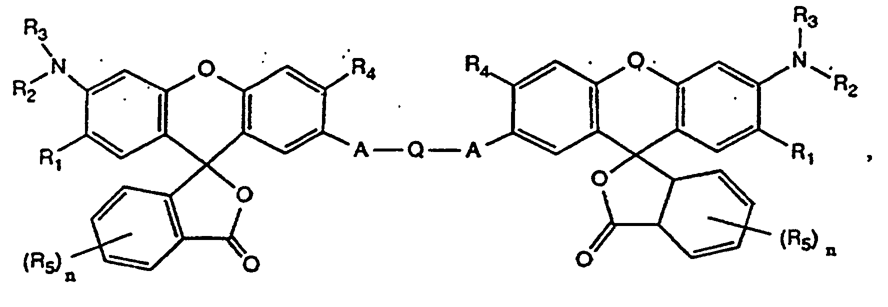 worin
R₁ Wasserstoff oder C₁-C₄-Alkyl;
R₂ und R₃ unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
R₂ und R₃ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
R₄ Wasserstoff, oder C₁-C₄-Alkyl;
R₅ Halogen; Nitro; C₁-C₄-Alkyl; C₁-C₄-Halogenalkyl; Amino; mono-C₁-C₄-Alkylamino; di-C₁-C₄-Alkylamino; oder COR₆
n 0; 1; 2; 3; oder 4;
R₆ Wasserstoff; Hydroxy; C₁-C₈-Alkyl; C₁-C₈-Alkoxy; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy,
A -(SO)O- oder -(CO)O-, welche über S bzw. C an das Fluorangerüst gebunden sind, und
Q einen gesättigten aliphatischen Rest, der durch Halogen substituiert sein kann, einen ungesättigten aliphatischen Rest oder einen gesättigten oder ungesättigten aliphatischen Rest, der durch Sauerstoffatome unterbrochen sein kann, wobei die Molekulargewichte dieser Reste zwischen 28 und 150 liegen, oder einen cycloaliphatischen oder araliphatischen Rest mit höchstens 10 Kohlenstoffatomen oder einen 2-Buten-1,4-diyl- oder Ethoxycarbonylethylenrest bedeutet.

2. Bislacton nach Anspruch 1, worin in Formel (1)
R₁ Wasserstoff oder Methyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten Pyrrolidin- oder Piperidin-Ring;
R₄ Wasserstoff oder Methyl;
n 0; 1; 2; 3; oder 4;
R₅ Halogen; Nitro; C₁-C₄-Alkyl; C₁-C₄-Halogenalkyl; mono-C₁-C₄-Alkylamino; di-C₁-C₄-Alkylamino; oder COR₆
A -(SO)O- oder -(CO)O- und
Q einen gesättigten oder ungesättigten aliphatischen Rest mit einem Molekulargewicht von 28 bis 150 oder einen cycloaliphatischen oder araliphatischen Rest mit höchstens 10 Kohlenstoffatomen,
bedeutet.

3. Bislacton nach Anspruch 1 oder 2, **dadurch gekennzeichnet, dass** Q in Formel (1) einen gesättigten oder ungesättigten aliphatischen oder cycloaliphatischen Rest bedeutet.

4. Bislacton nach Anspruch 3, **dadurch gekennzeichnet, dass** Q C₂-C₄-Alkylen bedeutet.

5. Bislacton nach einem der Ansprüche 1 bis 4, **dadurch gekennzeichnet, dass** n 0 bedeutet.

6. Bislacton nach Anspruch 1 der Formel (2) 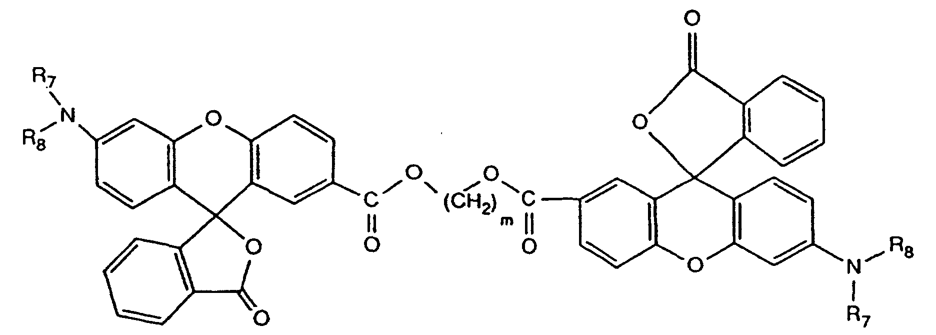 worin
R₇ und R₈ unabhängig voneinander C₁-C₅-Alkyl und
m 2 bis 4
bedeuten.

7. Verfahren zur Herstellung eines Bislactons der Formel (1) nach Anspruch 1, **dadurch gekennzeichnet, dass** man 2 Mol der Verbindung der Formel 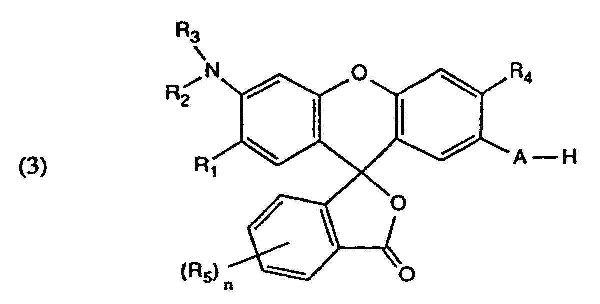 mit 1 Mol einer Dihalogenverbindung der Formel
(4) Hal-Q-Hal
umsetzt,
worin
R₁, R₂, R₃, R₄, R₅, A, Q und n die in Formel (1) angegebene Bedeutung haben und Hal Halogen bedeutet.

8. Verwendung der Bislactonverbindung nach einem der Ansprüche 1 bis 7 als Farbbildner in einem druck- oder wärmeempfindlichen Aufzeichnungsmaterial.

9. Verfahren zur Herstellung mikroverkapselter Farbbildner, **dadurch gekennzeichnet, dass** man mindestens eine Verbindung der Formel (1) nach einem oder mehreren der Ansprüche 1 bis 6 mikroverkapselt.

10. Verfahren zur Herstellung eines druckempfindlichen Aufzeichungsmaterials, **dadurch gekennzeichnet, dass** man auf einen ersten Träger Mikrokapseln enthaltend mindestens eine Verbindung der Formel (1) nach einem der Ansprüche 1 bis 6 aufträgt und auf den ersten Träger oder auf einen zweiten Träger, der in unmittelbaren Kontakt zu dem ersten Träger angeordnet ist, einen Entwickler aufträgt.

11. Verfahren zur Herstellung eines wärmeempfindlichen Aufzeichungsmaterials, **dadurch gekennzeichnet, dass** man auf einen Träger eine Mischung von Farbbildnern enthaltend mindestens eine Verbindung der Formel (1) nach einem der Ansprüche 1 bis 6 und einen Entwickler aufträgt.

12. Druck- oder wärmeempfindliches Aufzeichungsmaterial, enthaltend eine Mischung von Farbbildnern mit mindestens einer Verbindung der Formel (1) nach einem der Ansprüche 1 bis 6 und einen Entwickler.

## Claims

1. A bislactone of formula (1) 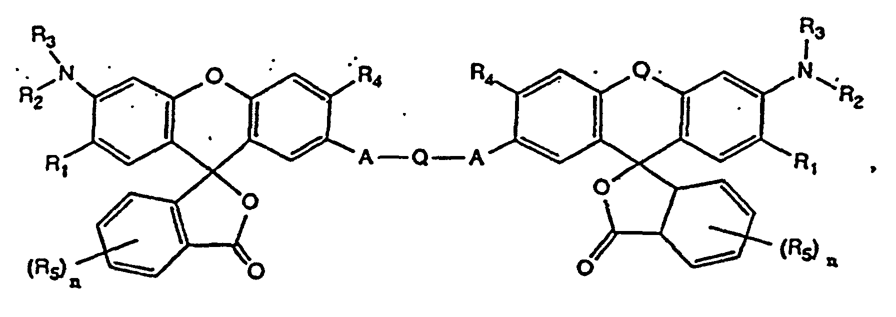 wherein
R₁ is hydrogen or C₁-C₄alkyl;
R₂ and R₃ are each independently of the other hydrogen; C₁-C₈alkyl; unsubstituted or C₁-C₄alkyl- or halogen-substituted C₄-C₇cycloalkyl; unsubstituted or C₁-C₄alkyl-, hydroxy- or halogen-substituted phenyl; phenyl-C₁-C₄alkyl; C₃-C₆alkenyl; C₁-C₄alkoxy; C₁-C₄alkoxy-C₁-C₄alkyl; 2-tetrahydrofuranyl; or
R₂ and R₃, together with the linking nitrogen atom, are a pyrrolidine, piperidine, morpholine, thiomorpholine or piperazine ring which is unsubstituted or substituted by C₁-C₄alkyl;
R₄ is hydrogen or C₁-C₄alkyl;
R₅ is halogen; nitro; C₁-C₄alkyl; C₁-C₄haloalkyl; amino; mono-C₁-C₄alkylamino; di-C₁-C₄alkylamino; or COR₆;
n is 0; 1; 2; 3; or 4;
R₆ is hydrogen; hydroxy; C₁-C₈alkyl; C₁-C₈alkoxy; C₁-C₈haloalkyl; or, unsubstituted or, in the phenyl radical, halogen-, C₁-C₄alkyl-, C₁-C₄haloalkyl- or C₁-C₄alkoxy-substituted, phenyl; phenyl-C₁-C₄alkyl or phenyl-C₁-C₄alkoxy;
A is -(SO)O- or -(CO)O- which are bonded to the fluoran structure by way of S or C, respectively; and
Q is a saturated aliphatic radical which may be substituted by halogen, an unsaturated aliphatic radical or a saturated or unsaturated aliphatic radical which may be interrupted by oxygen atoms, the molecular weights of those radicals being from 28 to 150, or a cycloaliphatic or araliphatic radical containing not more than 10 carbon atoms, or a 2-butene-1,4-diyl or ethoxycarbonylethylene radical.

2. A bislactone according to claim 1, wherein in formula (1)
R₁ is hydrogen or methyl;
R₂ and R₃ are each independently of the other hydrogen; C₁-C₅alkyl; or
R₂ and R₃, together with the linking nitrogen atom, are an unsubstituted pyrrolidine or piperidine ring;
R₄ is hydrogen or methyl;
n is 0; 1; 2; 3; or 4;
R₅ is halogen; nitro; C₁-C₄alkyl; C₁-C₄haloalkyl; mono-C₁-C₄alkylamino; di-C₁-C₄alkylamino; or COR₆;
A is -(SO)O- or -(CO)O- and
Q is a saturated or unsaturated aliphatic radical having a molecular weight from 28 to 150, or is a cycloaliphatic or araliphatic radical containing not more than 10 carbon atoms.

3. A bislactone according to either claim 1 or claim 2, wherein
Q in formula (1) is a saturated or unsaturated aliphatic or cycloaliphatic radical.

4. A bislactone according to claim 3, wherein
Q is C₂-C₄alkylene.

5. A bislactone according to any one of claims 1 to 4, wherein n is 0.

6. A bislactone according to claim 1 of formula (2) 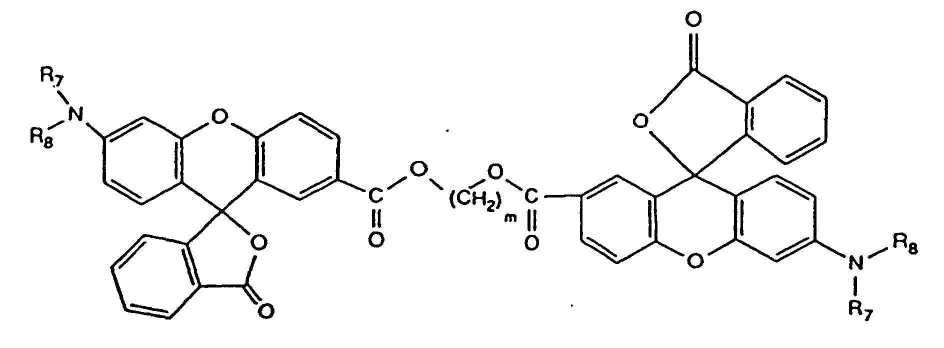 wherein
R₇ and R₈ are each independently of the other C₁-C₅alkyl, and
m is 2 to 4.

7. A process for the preparation of a bislactone of formula (1) according to claim 1, which comprises reacting 2 mol of the compound of formula 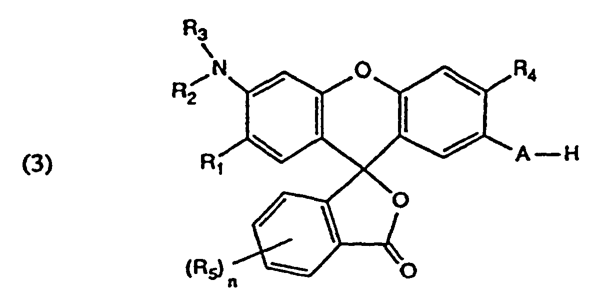 with 1 mol of a dihalo compound of formula
(4) Hal-Q-Hal
wherein
R₁, R₂, R₃, R₄, R₅, A, Q and n are as defined for formula (1), and Hal is halogen.

8. Use of the bislactone compound according to any one of claims 1 to 7 as colour former in a pressure-sensitive or heat-sensitive recording material.

9. A process for the preparation of a microencapsulated colour former, which comprises microencapsulating at least one compound of formula (1) according to one or more of claims 1 to 6.

10. A process for the preparation of a pressure-sensitive recording material, which comprises applying microcapsules comprising at least one compound of formula (1) according to any one of claims 1 to 6 to a first substrate and applying a developer to the first substrate or to a second substrate which is in direct contact with said first substrate.

11. A process for the preparation of a heat-sensitive recording material, which comprises applying a mixture of colour formers comprising at least one compound of formula (1) according to any one of claims 1 to 6 and a developer to a substrate.

12. A pressure-sensitive or heat-sensitive recording material comprising a mixture of colour formers with at least one compound of formula (1) according to any one of claims 1 to 6 and a developer.

## Revendications

1. Bislactone de formule (1) 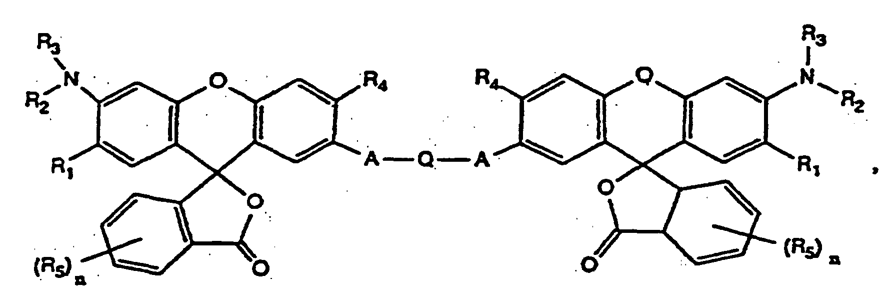 dans laquelle
R₁ représente un atome d'hydrogène ou un groupe alkyle en C₁ à C₄ ;
R₂ et R₃ représentent, indépendamment l'un de l'autre, un atome d'hydrogène ; un groupe alkyle en C₁ à C₈ ; un groupe cyclokalkyle en C₄ à C₇ non substitué ou substitué par un groupe alkyle en C₁ à C₄ ou un atome d'halogène ; un groupe phényle non substitué ou substitué par un groupe alkyle en C₁ à C₄, hydroxy ou un atome d'halogène ; un groupe phényl(alkyle en C₁ à C₄) ; alcényle en C₃ à C₆ ; alcoxy en C₁ à C₄ ; (alcoxy en C₁ à C₄) - (alkyle en C₁ à C₄) ; 2-tétrahydrofuranyle, ou bien
R₂ et R₃ représentent, conjointement avec l'atome d'azote auquel ils sont liés, un noyau pyrrolidine, pipéridine, morpholine, thiomorpholine ou pipérazine non substitué ou substitué par un groupe alkyle en C₁ à C₄ ;
R₄ représente un atome d'hydrogène ou un groupe alkyle en C₁ à C₄ ;
R₅ représente un atome d'halogène ; un groupe nitro ; un groupe alkyle en C₁ à C₄ ; un groupe halogénoalkyle en C₁ à C₄ ; un groupe amino ; un groupe monoalkylamino en C₁ à C₄ ; un groupe dialkylamino en C₁ à C₄ ; ou COR₆
n vaut 0 ; 1 ; 2 ; 3 ou 4 ;
R₆ représente un atome d'hydrogène ; un groupe hydroxy ; un groupe alkyle en C₁ à C₈ ; un groupe alcoxy en C₁ à C₈ ; un groupe halogénoalkyle en C₁ à C₈ ; un groupe phényle non substitué ou substitué sur le noyau phényle par un atome d'halogène, un groupe alkyle en C₁ à C₄, halogénoalkyle en C₁ à C₄, alcoxy en C₁ à C₄ ; un groupe phényl(alkyle en C₁ à C₄) ou phényl(alcoxy en C₁ à C₄),
A représente -(SO)O- ou -(CO)O-, lesquels sont liés par l'intermédiaire de l'atome S ou C au squelette fluorane, et
Q représente un groupe aliphatique saturé, qui peut être substitué par un atome d'halogène, un groupe aliphatique insaturé ou un groupe aliphatique saturé ou insaturé, qui peut être interrompu par des atomes d'oxygène, les masses moléculaires de ces groupes étant comprises entre 28 et 150, ou un groupe cycloaliphatique ou araliphatique contenant au maximum 10 atomes de carbone ou un groupe 2-butén-1,4-diyle ou éthoxycarbonyléthylène.

2. Bislactone selon la revendication 1, dans laquelle, dans la formule (1),
R₁ représente un atome d'hydrogène ou un groupe méthyle ;
R₂ et R₃ représentent, indépendamment l'un de l'autre, un atome d'hydrogène ; un groupe alkyle en C₁ à C₅ ; ou bien
R₂ et R₃ représentent, conjointement avec l'atome d'azote auquel ils sont liés, un noyau pyrrolidine ou pipéridine non substitué ;
R₄ représente un atome d'hydrogène ou un groupe méthyle ;
n vaut 0 ; 1 ; 2 ; 3 ou 4 ;
R₅ représente un atome d'halogène ; un groupe nitro ; un groupe alkyle en C₁ à C₄ ; un groupe halogénoalkyle en C₁ à C₄ ; un groupe monoalkylamino en C₁ à C₄ ; dialkylamino en C₁ à C₄ ; ou COR₆
A représente -(SO)O- ou -(CO)O- et
Q représente un groupe aliphatique saturé ou insaturé d'une masse moléculaire de 28 à 150 ou un groupe cycloaliphatique ou araliphatique contenant au maximum 10 atomes de carbone.

3. Bislactone selon la revendication 1 ou 2, **caractérisée en ce que**
Q dans la formule (1) représente un groupe aliphatique ou cycloaliphatique saturé ou insaturé.

4. Bislactone selon la revendication 3,
**caractérisée en ce que**
Q représente un groupe alkylène en C₂ à C₄.

5. Bislactone selon l'une des revendications 1 à 4, **caractérisée en ce que** n vaut 0.

6. Bislactone selon la revendication 1 de formule (2) 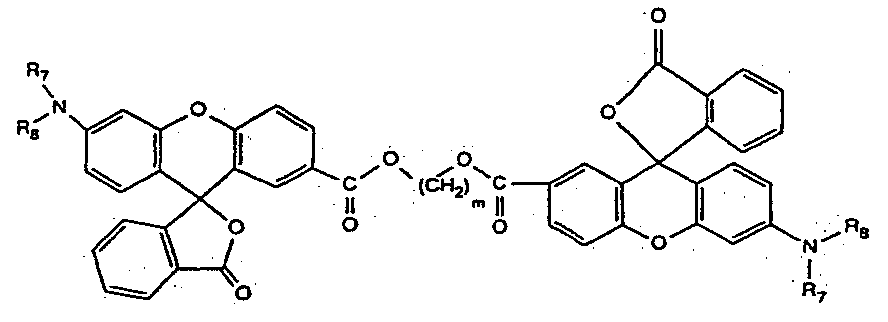 dans laquelle
R₇ et R₈ représentent, indépendamment l'un de l'autre, un groupe alkyle en C₁ à C₅ et
m vaut 2 à 4.

7. Procédé de préparation d'une bislactone de formule (1) selon la revendication 1, **caractérisé en ce que** l'on fait réagir 2 moles du composé de formule 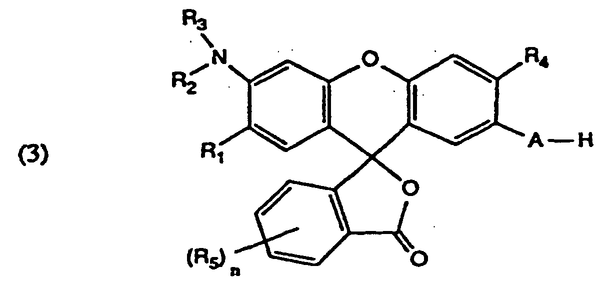 avec 1 mole d'un composé dihalogéné de formule
(4) Hal-Q-Hal
dans lesquelles
R₁, R₂, R₃, R₄, R₅, A, Q et n ont la signification mentionnée dans la formule (1) et Hal représente un atome d'halogène.

8. Utilisation du composé bislactone selon l'une des revendications 1 à 7 en tant que chromogène dans un matériau d'enregistrement sensible à la pression ou à la chaleur.

9. Procédé de préparation de chromogènes microencapsulés, **caractérisé en ce que** l'on microencapsule au moins un composé de formule (1) selon l'une ou plusieurs des revendications 1 à 6.

10. Procédé de fabrication d'un matériau d'enregistrement sensible à la pression, **caractérisé en ce que** l'on dépose, sur un premier support, des microcapsules contenant au moins un composé de formule (1) selon l'une des revendications 1 à 6 et, sur le premier support ou sur un deuxième support qui est en contact direct avec le premier support, un révélateur.

11. Procédé de fabrication d'un matériau d'enregistrement sensible à la chaleur, **caractérisé en ce que** l'on dépose sur un support un mélange de chromogènes, contenant au moins un composé de formule (1) selon l'une des revendications 1 à 6, et un révélateur.

12. Matériau d'enregistrement sensible à la pression ou à la chaleur, comprenant un mélange de chromogènes contenant au moins un composé de formule (1) selon l'une des revendications 1 à 6 et un révélateur.
